# EUROPÄISCHE PATENTSCHRIFT

(11) **EP 0 827 782 B1**
(45) Veröffentlichungstag und Bekanntmachung des Hinweises auf die Patenterteilung: **21.07.1999**
(21) Anmeldenummer: 97118764.6
(22) Anmeldetag: 25.06.1991
(51) Int. Cl.: B05B 11/00, G01F 11/02

(54) **Austragvorrichtung für Medien**
Fluid dispenser
Distributeur de fluides

(30) Priorität: 04.07.1990 DE 4021263
(43) Veröffentlichungstag der Anmeldung: 11.03.1998
(62) Teilanmeldung aus: 91912024.6
(73) Patentinhaber: Ing. Erich Pfeiffer GmbH, 78315 Radolfzell (DE)
(72) Erfinder: Graf, Lothar, 78239 Rielasingen-Worblingen (DE); Fuchs, Karl Heinz, 78315 Radolfzell (DE)
(74) Vertreter: Patentanwälte Ruff, Beier, Schöndorf und Mütschele

(56) Entgegenhaltungen:
- EP-A- 0 311 863
- WO-A-87/06141
- FR-A- 2 625 981
- US-A- 2 574 339
- US-A- 2 864 364
- US-A- 3 378 008
- US-A- 3 580 251
- US-A- 4 344 573

## Beschreibung

Die Erfindung betrifft eine Austragvorrichtung für fließfähige Medien, die meist der kosmetischen oder pharmazeutischen Anwendung dienen. Eine derartige Austragvorrichtung mit den Merkmalen gemäß Oberbegriff des Anspruchs 1 ist beispielsweise aus US-A-3 378 008 bekannt.

Die Anwendung derartiger Medien kann in einzelnen, aufeinanderfolgenden, stofflich oder im Volumen gleichen bzw. verschiedenen Chargen zweckmäßig sein. Hierzu können zwar Austragvorrichtungen mit Zählwerken für die ausgebrachten Chargenmengen vorteilhaft sein, jedoch ergibt sich hier eine verhältnismäßig komplizierte und raumaufwendige Ausbildung. Auch können mit üblichen Pumpendosierern aufeinanderfolgend gleich oder unterschiedlich große Chargen ausgebracht werden, wenn eine einstellbare Dosiereinrichtung vorgesehen ist. In diesem Fall kann jedoch die Anzahl der bereits ausgebrachten Chargen nicht festgestellt werden.

Der Erfindung liegt auch die Aufgabe zugrunde, Nachteile bekannter Ausbildungen zu vermeiden und insbesondere eine Austragvorrichtung zu schaffen, mit welcher auf einfache Weise genau bemessene Austragchargen ausgebracht werden können. Zweckmäßig ist die Austragvorrichtung zum Austrag durch eine manuelle Hub- bzw. Arbeitsbewegung ausgebildet.

Diese Aufgabe wird durch die Vorrictung gemäß Anspruch 1 gelöst.

Zweckmäßig weist die Austragvorrichtung als Austrageinheit mindestens eine Schubkolbenpumpe mit einem einzigen im wesentlichen formstabilen Pumpenzylinder bzw. Medien-Speicher und nicht zerstörbare Speicherkapseln für das Medium auf, so daß der Pumpenzylinder z.B. aus Glas gefertigt werden kann und sehr hohe hygienische Anforderungen erfüllt. Dies wird noch weiter verbessert, wenn die befüllte Austragvorrichtung das Medium nur in mindestens einem solchen Behälter enthält, der gleichzeitig den Pumpenzylinder für die Führung eines Pumpkolbens bildet und diesem in Ausgangsstellung im Abstand gegenüberliegend am Boden dicht geschlossen ist, nämlich keine Einlaß- oder Ansaugöffnung aufweist.

Bei der beschriebenen oder einer anderen Ausbildung der Austragvorrichtung kann auch vorteilhaft vorgesehen sein, daß wenigstens ein Teil des Kolbenstößels einer Kolbeneinheit in einer Ausgangsstellung im wesentlichen vollständig von der Kolbeneinheit getrennt ist und erst durch eine Hubbewegung mit dem Pumpkolben gekuppelt werden kann. Befindet sich hierbei der Pumpkolben bereits innerhalb des Pumpenzylinders, so kann er einen dichten Verschluß des in der Pumpenkammer gespeicherten Mediums bilden. Der Verschluß wird beim Ankuppeln des Kolbenstößels selbsttätig durch Zerstörung geöffnet und dabei gleichzeitig die Leitungsverbindung zwischen der Pumpenkammer und einem Auslaßkanal bzw. einer Auslaßöffnung hergestellt. Da das Medium bei einer solchen Ausbildung völlig dicht versiegelt in der Pumpenkammer gespeichert werden kann, können sehr große Lagerzeiten erreicht werden. Außerdem kann der Kupplungsvorgang so gestaltet sein, daß er eine Sicherung gegen unbefugte Benutzung, z.B. durch Kinder, bildet.

Dadurch wird praktisch für jeden Austrag ein neuer, versiegelter Behälter angebrochen, der z.B. ein Speichervolumen von einer Größenordnung von nur etwa 0,1 ml zu haben braucht. Eine solche Austragvorrichtung kann sehr kleine Abmessungen haben.

Die Austragvorrichtung kann sehr einfach ausgebildet werden. Z.B. weist sie zwei im wesentlichen einteilige, teleskopartig ineinandergreifende Kappen auf, deren voneinander abgekehrte, freiliegende Stirnwände unmittelbar Druck-Handhaben zur Hubbetätigung bilden. Über eine dieser Stirnwände steht zweckmäßig ein den Kolbenstößel tragender Austragstutzen vor, während die andere Stirnwand die zugehörige Endbegrenzung der Austragvorrichtung bildet. Die zugehörige Kappe weist im Inneren und nach außen vollständig verdeckt mindestens eine Aufnahme zur Steckbefestigung eines Speicher- bzw. Zylinderbehälters auf.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:

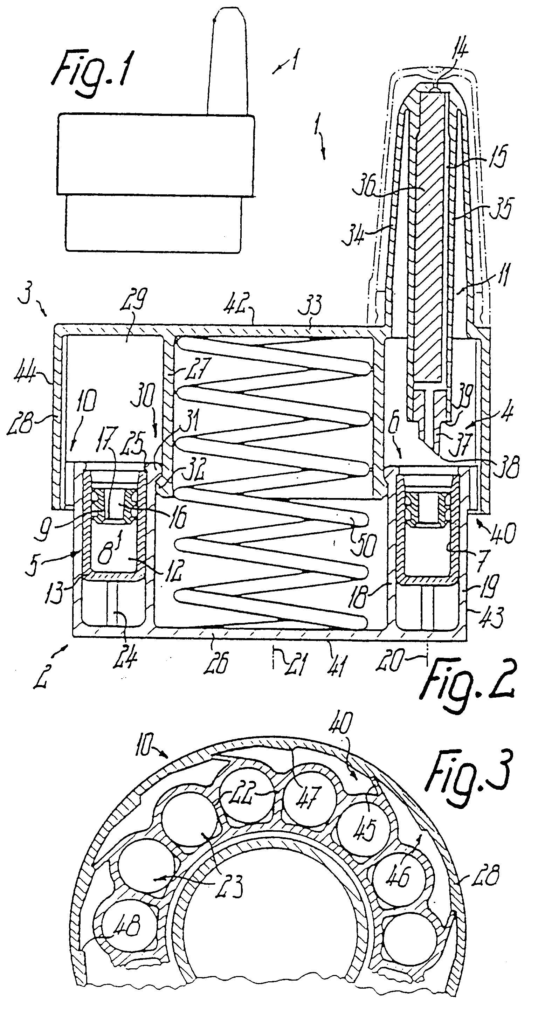

- Fig. 1: eine erfindungsgemäße Austragvorrichtung in Ansicht und etwa natürlicher Größe,
- Fig. 2: die Austragvorrichtung, vergrößert im Axialschnitt und
- Fig. 3: einen Querschnitt durch die Anordnung gemäß Fig. 2.

Die Austragvorrichtung 1 ist an ihrer Außenseite im wesentlichen von zwei Gehäusekörpern 2, 3 begrenzt, die nach Art einer flachen Dose mit Deckel ineinandergreifen und im Innern vollständig nach außen abgedeckt eine Pumpenanordnung 4 mit einer Vielzahl von Pumpen 6 aufnehmen.

Jeder Pumpe 6 ist ein Zylinder 7 einer Zylinderanordnung 5 zugeordnet, der mit einem Pumpenkolben 9 einer Kolbeneinheit 8 eine Montage-Baueinheit bildet. Mit einer Folgeschaltung 10 kann jede Pumpe aus einer Ruhelage in eine Arbeitslage überführt werden, in welcher sie zur Betätigung mit einem Stößel 11 etwa achsgleich ausgerichtet ist und ihre durch einen Behälter 13 gebildete Pumpenkammer 12 über einen Auslaßkanal 15 durch eine Auslaßöffnung 14 unmittelbar ins Freie entleert werden kann.

Zu diesem Zweck weist jede Pumpe 6 in einer achsgleichen Durchgangsöffnung 16 ihres hülsenförmigen Pumpkolbens 9 einen Verschluß 17 in Form beispielsweise einer sehr dünnen Membran auf, die mit ihrem Rand an der Innenfläche der Durchgangsöffnung 16 dicht befestigt ist. Der Pumpkolben 9 liegt in Ausgangslage vollständig innerhalb des Behälters 13 benachbart zu dessen auf voller Innenweite des Zylinders 7 offenem Ende und bildet mit dem Verschluß 17 einen die Pumpenkammer 12 dicht nach außen verschließenden Verschlußstopfen. Der Pumpkolben 9 weist z.B. drei axial hintereinander liegende, einteilig mit der Kolbenhülse ausgebildete Dichtlippen auf, die ausschließlich zu seiner Halterung und Führung an der Zylinderlaufbahn dienen.

Boden und Mantel jedes Behälters 13 sind einteilig ausgebildet, und der Verschluß 17 liegt bei dem dem Boden zugekehrten Ende des Pumpkolbens 9 bzw. der Durchgangsöffnung 16. Alle Pumpen 6 sind annähernd achsparallel zueinander, zum Stößel 11 und zum jeweiligen Gehäusekörper 2 bzw. 3 so angeordnet, daß ihre Enden jeweils etwa in einer gemeinsamen Ebene liegen.

Der im wesentlichen zylindrische und in der Außenweite kleinere Grund- bzw. Gehäusekörper 2 weist zwei annähernd koaxial ineinanderliegende Mäntel 18, 19 auf, die über etwa radiale Stege 22 einteilig miteinander verbunden sind und zwischen jeweils zwei Stegen eine Halterung 23 für eine Pumpe 5 bzw. einen Behälter 13 bilden. Die Achsen 20 der Halterungen 23 liegen in einem Kranz um die gemeinsame Achse 21 des Gehäuses, von der die Mittelachse des Stößels 11 denselben Abstand hat. Am Boden jeder Halterung 23, die eine eng an den Behälter 13 angepaßte, axiale Stecköffnung bildet, ist eine Stütze 24, z.B. mindestens eine Rippe, vorgesehen, an welcher der zugehörige Behälter 13 mit seinem Boden im Abstand vom Boden der Stecköffnung abgestützt ist.

Die offene Stirnfläche des jeweiligen Behälters 13 wird von einer ringwulstförmigen Rast 25 des Halterungsmantels übergriffen, wodurch sich eine spielfreie Schnappbefestigung des Behälters 13 ergibt, der annähernd bis an die offene, innere Stirnseite des Gehäusekörpers 2 reicht. An der äußeren Stirnseite ist der Gehäusekörper 2 mit einer annähernd ebenen Stirnwand 26 verschlossen. In Ausgangslage ragt der Gehäusekörper 2 über den größten Teil seiner Länge aus dem Gehäusekörper 3. Dieser weist ebenfalls zwei koaxial ineinanderliegende Mäntel 27, 28 auf, zwischen denen ein Ringraum 29 für den drehbaren und axial verschiebbaren Eingriff des ringförmigen Halterungsvorsprunges des Gehäusekörpers 2 begrenzt ist.

Die Führung 30 ist im wesentlichen nur durch die inneren Mäntel 18, 27 gebildet, wobei eine Wulst 31 am inneren Ende des Mantels 18 am Innenumfang des Mantels 27 und eine Wulst 32 des inneren Endes des Mantels 27 am Innenumfang des Mantels 18 gleitet. Diese Wulste 31, 32 bilden gleichzeitig Schnappglieder zur einfachen Montageverbindung der Gehäusekörper 2, 3 und die einzige Axialsicherung zur Festlegung der Ausgangsstellung, in welcher sie mit Ringschultern aneinander anliegen. Die Mäntel 27, 28 sind ebenfalls einteilig mit einer im wesentlichen ebenen Stirnwand 33 ausgebildet, die mit der Stirnwand 26 die Endbegrenzungen des Gehäuses bildet und über die nur ein den Stößel 11 versenkt aufnehmender und in seiner Endfläche die Auslaßöffnung 14 aufweisender Stutzen 34 vorsteht.

Ein äußerer Stutzenmantel schließt einteilig an die Stirnwand 33 an und geht bei seinem freien Ende in einen innen liegenden Stößelmantel 35 über, der die Stirnwand 33 berührungsfrei durchsetzt und in den Ringraum 29 ragt. Innerhalb des Stößelmantels 35 ist ein schaftförmiger Stößelkern 36 angeordnet, der mit einem in der Weite reduzierten Vorsprung 37 über das innere Ende des Stößelmantels 35 vorsteht und mit seinem anderen Ende einen Bestandteil einer Dralleinrichtung für die Auslaßöffnung 14 bilden kann. Der Auslaßkanal 15 durchsetzt den Vorsprung 37 in seiner Endfläche, die zur Bildung einer Spitze 38 abgeschrägt ist.

Im Übergangsbereich zum Vorsprung 37 bildet der Stößelkern 36 mit dem Stößelmantel 35 eine ringförmige Schulter 39, die abgedichtet an der zugehörigen, ebenen Stirnfläche des jeweiligen Pumpkolbens 9 anliegen kann. Der Vorsprung 37 ist eng an die Durchgangsöffnung 16 angepaßt und kann wie diese zylindrisch oder konisch sein. Das Ende des Vorsprunges 37 liegt in Ausgangslage in geringem Abstand von der inneren Stirnseite des Gehäusekörpers 2.

Die beiden Gehäusekörper 2, 3 sind durch ein durch die Folgeschaltung 10 gebildetes Gesperre 40 nur in einer Richtung gegeneinander verdrehbar. Sie bilden mit den äußeren Stirnflächen ihrer Stirnwände 26, 33 jeweils eine Druck-Handhabe 41, 42 und mit ihrem Außenumfang jeweils eine Dreh-Handhabe 43 bzw. 44; in Pumphubendlage liegt der Gehäusekörper 2 und damit dessen Handhabe 43 im wesentlichen vollständig innerhalb des Gehäusekörpers 3. Am Außenumfang des Mantels 19 sind zungenförmig frei und schräg entgegen der Drehrichtung des Gehäusekörpers 2 vorstehende Rastglieder 45 verteilt vorgesehen, denen am Innenumfang des Mantels 28 eine über dessen gesamte Länge durchgehende Zahnung 46 mit einer Teilung zugeordnet ist, die der Teilung der Halterungen 23 entspricht. Die Zahnung 46 bildet nach innen vorstehende Zähne bzw. Rastglieder 47, die jeweils an einer Flanke von einer flach ansteigenden Gleitfläche und an der anderen Flanke von einer annähernd radial zur Achse 21 liegenden Sperrschulter 48 begrenzt sind.

Die einteilig mit dem Mantel 19 ausgebildeten Rastglieder 45 sind nur an einem kurzen, an das innere Ende anschließenden Abschnitt des Gehäusekörpers 2 vorgesehen, so daß sie in Ausgangslage vollständig innerhalb des Gehäusekörpers 3 liegen.

In einer Raststellung der Folgeschaltung 10 bzw. des Gesperres 40 liegt der Vorsprung 37 achsgleich zu einer Pumpe 6. Werden nunmehr die Gehäusekörper 2, 3 entgegen der Kraft einer Rückstellfeder 50 zusammengedrückt, so tritt der Vorsprung 37 in den Pumpkolben 9 ein, durchsticht den Verschluß 17 und nimmt dann über die Schulter 39 den Pumpkolben 9 bis zur Anlage am Boden des Behälters 13 mit, da der Vorsprung 37 im wesentlichen nicht über das innere Ende des Pumpkolbens 9 vorsteht. Während dieser Pumphubbewegung wird der Inhalt der Pumpenkammer 12 durch den Auslaßkanal 15 und die Auslaßdüse vollständig ausgetragen.

Durch die beschriebene Ausbildung ist der Stößel 11 geringfügig federnd auslenkbar, so daß er sich beim Kuppeln von selbst genau gegenüber dem Pumpkolben 9 ausrichten kann. Werden danach die Handhaben 41, 42 wieder freigegeben, so wird der Vorsprung 37 wieder aus dem Behälter 13 herausgezogen. Durch die sägezahnförmige Ausbildung der Kolbenlippen ist hierbei die Reibung gegenüber dem Behälter 13 so groß, daß sich der Stößel 11 bereits zu Beginn des Rückhubes wieder vom Pumpkolben 9 löst und dieser in seiner Endlage stehen bleibt.

In Ausgangslage können dann die beiden Gehäusekörper 2, 3 um einen Schaltschritt der Folgeschaltung 10 gegeneinander verdreht werden, wonach der Stößel 11 fluchtend zu einer weiteren Pumpe 6 steht. Während der Hubbewegungen bleibt die Drehsperre aufrechterhalten, weil die Rastglieder 45 an den Sperrschultern 48 gleiten.

Durch die magazinartige Ausbildung des Gehäuseteiles 2 können auch Pumpeneinheiten mit unterschiedlichen Wirkstoffen oder Wirkstoffkonzentrationen aufgenommen werden, und außerdem läßt sich das Magazin nach Trennen der Gehäuseteile 2, 3 auch wieder mit frischgefüllten Pumpeneinheiten nachrüsten.

Die Rückstellfeder 50 liegt seitlich benachbart und achsparallel zu den Pumpen 6 in der Achse 21. Sie ist im Mantel 27 zentriert und stützt sich an den Innenseiten der Stirnwände 26, 33 ab. Dadurch kann die außerhalb der Pumpenachsen liegende Rückstellfeder 50 die Pumpen 6 an einem oder beiden Enden überragen. Die Austragvorrichtung 1 läßt sich sehr einfach herstellen, montieren sowie handhaben und weist eine sehr kompakte Ausbildung auf. Der Auslaßkanal 15 ist im dargestellten Ausführungsbeispiel ventilfrei bzw. durchgehend offen ausgebildet, kann aber auch mit einem z.B. druckabhängig arbeitenden Auslaßventil versehen sein.

Die dargestellte Austragvorrichtung kann auch mit Merkmalen einer im folgenden erläuterten Austragvorrichtung so kombiniert werden, daß der jeweilige Pumpkolben 9 über zwei oder mehr aufeinanderfolgende Teilstrecken seines Gesamthubes jeweils anschlagbegrenzt betätigt werden kann, wonach der nächste Teilhub ausgeführt werden kann.

Bei einer solchen Pumpe wäre nur eine einzige Pumpe mit Pumpkolben und Pumpenzylinder vorgesehen, die in einer Achse liegen. Der Behälter könnte eine gegenüber seinem Durchmesser vielfach größere Länge aufweisen, so daß die Pumpenkammer durch schrittweises, jedoch rückstellungsfreies Einfahren des Pumpkolbens entleert werden kann.

## Patentansprüche

1. Austragvorrichtung für Medien mit einer Austrageinheit (6, 6a), die zum Austrag mindestens einer Austragcharge durch eine manuelle Hubbetätigung ausgebildet ist,
a) mit einem Gehäuse,
b) in dem ein Kolbenstößel (11) angeordnet ist,
c) der wenigstens einen Auslaßkanal (15) abgrenzt,
d) mit einem Medienbehälter (13) für die Charge mit einer Öffnung, die dem Kolbenstößel (11) zugewandt ist,
e) wobei ein Medienbehälter-Verschluß (17) die Öffnung des Medienbehälters (13) dicht verschließt
f) und mit einem gegen den MedienbehälterVerschluß (17) gerichteten, in den Kolbenstößel eingesetzten Durchstechelement (37),
g) das beim Gegeneinanderdrücken des Medienbehälters (13) und des Kolbenstößels (11) den Medienbehälter-Verschluß (17) durchdringen und
h) das Medium über den Auslaßkanal (15) und eine Auslaßöffnung (14) austreten lassen kann, dadurch gekennzeichnet,
i) daß das Durchstechelement (37) mit der im Gehäuse (3) vorgesehenen, düsenformigen Auslaßöffnung (14) über eine Dralleinrichtung in Verbindung steht.

2. Austragvorrichtung nach Anspruch 1, dadurch gekennzeichnet, daß die Auslaßöffnung (14) an einem Auslaßstutzen (34, 34a) vorgesehen ist, wobei vorzugsweise wenigstens eine Betätigungsschulter (42, 42a) seitlich von dem Auslaßstutzen (34, 34a) vorgesehen ist, und im Inneren des Auslaßstutzens (34, 34a) der Kolbenstößel (11) angeordnet ist.

3. Austragvorrichtung nach Anspruch 1 oder 2, dadurch gekennzeichnet, daß der Verschluß (9) aus einem Stopfen besteht, der an der Innenwand (7) des Medienbehälters (13) gegenüber dieser beweglich anliegt und eine zentrale Membran (17) aufweist, die von dem Durchstechelement (37) durchstechbar ist, wobei vorzugsweise das Durchsiechelement (37) nicht wesentlich über die behälterseitige Innenbegrenzung des Pfropfens vorsteht.

4. Austragvorrichtung nach einem der vorhergehenden Ansprüche, dadurch gekennzeichnet, daß das Durchstechelement (37) zur Bildung des Auslaßkanals (15) hohl und vorzugsweise zur Bildung einer Spitze (38) abgeschrägt ist und sich insbesondere in der Achse des Kolbenstößels (11) zur Auslaßöffnung (14) erstreckt.

5. Austragvorrichtung nach einem der vorhergehenden Ansprüche, dadurch gekennzeichnet, daß der Medienbehälter (13) in einer hülsenartigen Halterung (23) aufgenommen ist, die eine Druck-Handhabe (41, 42) zur Betätigung der Hubbewegung bildet.

6. Austragvorrichtung nach einem der vorhergehenden Ansprüche, gekennzeichnet durch eine Axialsicherung zur Festlegung der Ausgangsstellung des Medienbehälters (13) und des Kolbenstößels (11) zueinander, wobei vorzugsweise zur Axialsicherung Schnappglieder vorgesehen sind und insbesondere eine Wulst (31) an einer Halterung (18) des Medienbehälters (13) mit einer Wulst (32) im Gehäuse (3) zusammenwirkt.

7. Austragvorrichtung nach Anspruch 6, dadurch gekennzeichnet, daß in der Ausgangsstellung zwischen dem Kolbenstößel (11) und dem Medienbehälter-Verschluß (9) ein Abstand vorgesehen ist.

8. Austragvorrichtung nach einem der vorhergehenden Ansprüche, dadurch gekennzeichnet, daß der Kolbenstößel (11) von dem als Kolben wirkenden Medienbehälter-Verschluß (9) getrennt ist und erst durch eine Hubbewegung damit gekuppelt wird, wobei er insbesondere eine Stirnfläche (39) aufweist, die anschließend an das Durchdringen des Durchstechelementes (37) durch den Verschluß (17) diesen als Kolben bis zur Anlage am Boden des Medienbehälters (13) mitnimmt.

9. Austragvorrichtung nach einem der vorhergehenden Ansprüche, dadurch gekennzeichnet, daß die Hubbewegung über zwei oder mehr aufeinanderfolgende Teilstrecken des Gesamthubes jeweils anschlagbegrenzt und rückstellungsfrei betätigbar ist.

10. Austragvorrichtung nach einem der vorhergehenden Ansprüche, dadurch gekennzeichnet, daß sie zum Austrag pharmazeutischer Medien ausgebildet ist, wobei vorzugsweise der Medienbehälter (13) ein Volumen von ca. 0,1 ml hat.

## Claims

1. Dispenser for media having a dispensing unit (6, 6a) constructed for dispensing at least one dispensing charge by a manual stroke actuation,
a) having a casing,
b) in which a plunger ram (11) is located,
c) which defines at least one outlet passage (15),
d) having a medium container (13) for the charge with an opening facing the plunger ram (11),
e) in which a medium container closure (17) tightly seals the opening of the medium container (13),
f) and having a perforating element (37), inserted in the plunger ram and directed against the medium container closure (17),
g) which on pressing against one another the medium container (13) and plunger ram (11) penetrates the medium container closure (17) and
h) can permit the outflow of the medium via the outlet passage (15) and an outlet port (14),
characterized in that
i) the perforating element (37) is connected by means of a whirling device to the nozzle-like outlet port (14) located in the casing (3).

2. Dispenser according to claim 1, characterized in that the outlet port (14) is located on an outlet connection (34, 34a) and preferably at least one actuating shoulder (42, 42a) is provided laterally of the outlet connection (34, 34a) and the plunger ram (11) is located in the interior of the outlet connection (34, 34a).

3. Dispenser according to claim 1 or 2, characterized in that the closure (9) comprises a plug, which on the inner wall (7) of the medium container (13) movably engages against the latter and has a central diaphragm (17), which is perforatable by the perforating element (37), which preferably does not project significantly over the container-side inner boundary of the plug.

4. Dispenser according to one of the preceding claims, characterized in that the perforating element (37) is hollow for forming the outlet passage (15) and is preferably bevelled for forming a tip (38) and in particular extends in the axis of the plunger ram (11) towards the outlet port (14).

5. Dispenser according to one of the preceding claims, characterized in that the medium container (13) is received in a sleeve-like holder (23), which forms a pressure handle (41, 42) for actuating the stroke movement.

6. Dispenser according to one of the preceding claims, characterized by an axial securing means for fixing the starting position of the medium container (13) and the plunger ram (11) with respect to one another and preferably snap-action members are provided for axial securing purposes and in particular a collar (31) on a holder (18) of the medium container (13) cooperates with a collar (32) in the casing (3).

7. Dispenser according to claim 6, characterized in that in the starting position a spacing is provided between the plunger ram (11) and the medium container closure (9).

8. Dispenser according to one of the preceding claims, characterized in that the plunger ram (11) is separated from the medium container closure (9) acting as a plunger and is only coupled thereto by a stroke movement and it in particular has an end face (39), which following the penetration by the penetrating element (37) of the closure (17) carries along the latter as a plunger up to engagement on the bottom of the medium container (13).

9. Dispenser according to one of the preceding claims, characterized in that the stroke movement is actuatable over two or more successive portions of the total stroke in each case in stop-limited and restoration-free manner.

10. Dispenser according to one of the preceding claims, characterized in that it is constructed for the dispensing of pharmaceutical media and preferably the medium container (13) has a volume of approximately 0.1 ml.

## Revendications

1. Distributeur de fluides avec une unité de distribution (6, 6a) formée pour la distribution d'au moins une charge de distribution par une action de levage manuelle
a) avec un boîtier,
b) dans lequel est placé un coulisseau à piston (11),
c) qui limite au moins un canal de sortie (15),
d) avec un réservoir de fluides (13) pour la charge, avec une ouverture tournée vers le coulisseau à piston (11),
e) un bouchon de réservoir de fluides (17) fermant de façon étanche l'ouverture du réservoir de fluides (13),
f) et avec un élément de perforation (37) inséré dans le coulisseau à piston (11) et dirigé contre le bouchon du réservoir de fluides (17),
g) qui traverse le bouchon du réservoir de fluides (17) lors de la pression mutuelle du réservoir de fluides (13) et du coulisseau à piston (11), et
h) pouvant laisser sortir le fluide par le canal de sortie (15) et une ouverture de sortie (14), **caractérisé en ce que**
i) l'élément de perforation (37) est raccordé à l'ouverture de sortie (14) en forme de buse prévue dans le boîtier (3) par un dispositif de torsion.

2. Distributeur de fluides selon la revendication 1, **caractérisé en ce** que l'ouverture de sortie (14) est prévue sur une tubulure de sortie (34, 34a), au moins une épaule d'actionnement (42, 42a) étant prévue de préférence latéralement par rapport à la tubulure de sortie (34, 34a) et étant placée à l'intérieur de la tubulure de sortie (34, 34a) du coulisseau à piston (11).

3. Distributeur de fluides selon la revendication 1 ou 2, **caractérisé en ce que** le bouchon (9) est composé d'un tampon prenant appui de manière mobile contre la paroi intérieure (7) du réservoir de fluides (13) et présentant une membrane centrale (17) perforable par l'élément de perforation (37), ce dernier ne dépassant, de préférence, pas beaucoup de la limite intérieure du côté du réservoir.

4. Distributeur de fluides selon l'une des revendications précédentes, **caractérisé en ce que** l'élément de perforation (37) est creux pour la formation du canal de sortie (15) et de préférence biseauté pour la formation d'une pointe (38) et qu'il s'étend notamment dans l'axe du coulisseau à piston (11) vers l'ouverture de sortie (14).

5. Distributeur de fluides selon l'une des revendications précédentes, **caractérisé en ce que** le réservoir de fluides (13) est logé dans un support (23) en forme de douille qui forme une prise de pression (41, 42) pour l'actionnement du mouvement de levage.

6. Distributeur de fluides selon l'une des revendications précédentes, **caractérisé par** une sécurité axiale pour la fixation de la position initiale du réservoir de fluides (13) et du coulisseau à piston (11) l'un par rapport à l'autre, des éléments à déclic étant de préférence prévus pour la sécurité axiale et notamment un bourrelet (31) sur un support (18) du réservoir de fluides (13) coopérant avec un bourrelet (32) du boîtier (3).

7. Distributeur de fluides selon la revendication 6, **caractérisé en ce qu'**un écart est prévu entre le coulisseau à piston (11) et le bouchon du réservoir de fluides (9) en position initiale.

8. Distributeur de fluides selon l'une des revendications précédentes, **caractérisé en ce que** le coulisseau à piston (11) est séparé du bouchon de réservoir de fluides (9) agissant comme piston et qu'il n'est coupé à celui-ci que par un mouvement de levage, le coulisseau présentant notamment une surface frontale (39) qui, suite à la pénétration de l'élément de perforation (37) dans le bouchon (17), entraîne celui-ci comme piston jusqu'à prendre appui sur le fond du réservoir de fluides (13).

9. Distributeur de fluides selon l'une des revendications précédentes, **caractérisé en ce que** le mouvement de levage peut être commandé sur deux ou plusieurs sections partielles successives de la course totale, respectivement limité par butée et sans être remis en position initiale.

10. Distributeur de fluides selon l'une des revendications précédentes, **caractérisé en ce qu**'il est formé pour la distribution de fluides pharmaceutiques, le réservoir de fluides (13) ayant de préférence un volume d'environ 0,1 ml.
